# EUROPEAN PATENT SPECIFICATION

(11) **EP 2 168 622 B1**
(45) Date of publication and mention of the grant of the patent: **29.07.2015**
(21) Application number: 08772913.3
(22) Date of filing: 29.05.2008
(51) Int. Cl.: A61M 5/50, A61M 5/178, A61M 5/31, A61J 3/00

(54) **SINGLE USE SELF-BLOCKING SYRINGE WITH POSSIBILITY TO PERFORM DRUG RECONSTITUTION AND BLOOD ASPIRATION TEST**
EINWEGSPRITZE MIT MÖGLICHKEIT REKONSTITUTION UND BLUTANSAUGTEST DURCHZUFÜHREN UND SELBSTBLOCKIERUNG NACH DER INJEKTION
SERINGUE POUR USAGE UNIQUE AVEC POSSIBILITÉ DE RECONSTITUTION DE MÉDICAMENT ET TEST D'ASPIRATION DU SANG

(30) Priority: 01.06.2007 CN 200720152414 U
(43) Date of publication of application: 31.03.2010
(73) Proprietor: Wuxi Yushou Medical Appliances Co., Ltd., Dongbeitang, Xishan District Wuxi Jiangsu 214191 (CN)
(72) Inventor: FENG, Zhong, Jiangsu 214191 (CN)
(74) Representative: Stolmár & Partner Patentanwälte PartG mbB
(86) International application number: PCT/CN2008/001054
(87) International publication number: WO 2008/148299

(56) References cited:
- EP-A1- 1 066 847
- WO-A1-01/80932
- WO-A1-01/80932
- WO-A1-2005/058397
- CN-Y- 2 817 870
- CN-Y- 2 817 870
- CN-Y- 2 873 231
- CN-Y- 2 873 231
- CN-Y- 201 055 566
- US-A- 5 037 393
- US-A- 5 059 181
- US-B1- 6 283 941

## Description

### TECHNICAL FIELD

The present invention relates generally to a syringe for medical treatment, and more particularly to, a self-destructive syringe with drug dissolved for once.

### BACKGROUND OF THE INVENTION

At present, the syringes widely used in the medical institutions in a native or foreign country are still conventional type of the syringes for one-time use. It is absolutely possible that the conventional types of the syringes are consciously reused by the operators, and if so, the possibility of disease cross infection will be greatly increased. In recent years, for resolving the above problems, many companies or individuals have designed a variety of self-destructive syringes. For example, two types of the syringes with the self-destructive means which can be used for dissolving drugs are disclosed in ZL02265318.X and ZL99247769.7 (Yushou Medical Instrument Ltd. of Wuxi City, China).The syringe in ZL02265318.X may be reused many times if the protecting sheet 6 is not removed when the syringe is used to dissolve the drug or to inject the liquid drug. In addition, although ZL99247769.7 also discloses the syringe provided with a stroke-limiting protecting sheet, the stroke-limiting protecting sheet may be easily pierced during mounting and packaging, transporting or before using. Actually, in the present production of the syringes, the stroke-limiting protecting sheet is also added. Although the syringe is used to dissolve the drug, if the stroke-limiting protecting sheet is not removed, the syringe may be also reused many times. To be suitable for diluting the powders such as freeze-dried vaccines, a restricted syringe overcomes the shortcoming of re-use and it can only be drawn and injected two times by means of mechanical construction control thereby preventing the syringes from abusing, as described in PCT Application Publication WO01/80932A1. However, the construction of this syringe is complicated, and the production cost is very high. Besides, although the backstreaming of blood can be observed, the moving stroke from the piston and pushing rod for observing the backstreaming of blood to the locking element is relatively long and the size is not defined. As shown in Fig. 10G, the syringe is inserted into the human body after expelling the air, the backstreaming of blood has the volume in 0.05 ml because a 0.05 ml syringe may be used for injection of 0.05 ml vaccines.

CN 2 873 231 Y describes a disposal self-destroying injector consisting of a protecting sleeve, a needle, a hub, a pushing pole, a barrel and a stopping steel piece. The injector is characterized in that the hub s provided with a convex edge and that the pushing pole is provided with a sealing ring, a front movable portion, a front spacing step, a ratchet groove, a back movable portion and a back spacing step and that the barrel is provided with a protecting convex edge and that the stopping steel piece is provided with a ratchet and a stopping stab.

US 6 283 941 B1 describes a single-use syringe having a rod-like plunger comprising a plurality of frusto-conical or bead-like ratchet teeth. A radially resilient, spring locking clip dangles on the ratchet portion of the plunger.

US 5 037 393 A discloses a non-reusable syringe comprising a syringe body having a lower extremity closed by a base provided with a cone to receive a needle and a shaft terminated by a piston sliding tightly in the syringe body. A resilient flexible plate having a diameter greater than the inner diameter of the syringe body and thus inducing through its curvature a weak frictional force enabling the displacement of the shaft in a first direction and a strong frictional force opposing displacement of the shaft in the other direction is mounted in the shaft. To avoid attempted reuse of the syringe, a zone of weak resistance to breakage by traction is provided on the shaft. This zone breaks under traction lower than the strong frictional force, but greater than the weak frictional force.

CN 2 817 870 Y discloses a medical syringe, more specifically, the utility model discloses a disposable self-destruction syringe, the aim of which is to provide a disposable self-destruction syringe which has few residual quantities and starts self-destruction when physic liquor is injected. The disposable self-destruction syringe comprises a needle tip, a cylinder body, a core rod, a return stopping piece and a sealing plug. The utility model is characterized in that the core rod is provided with a ratchet groove, a return limiting step, an installing hole and a repeatedly moved part, and the return stopping piece is provided with a return stopping spine and a ratchet. The utility model is used for immunization and insulin injection, and has the advantages of few residual quantities, reliable and convenient use, and self-destruction after disposable injection.

In WO 2005/058397 A1 a disposable syringe is disclosed. Withdrawal of a plunger handle in the direction of arrow B to fill the syringe causes a moveable safety bobbin to lock, and shaft slides in the direction of arrow B relative to safety bobbin and washer. Subsequent depression of plunger handle in the direction of arrow A causes safety bobbin to move along barrel, from which position it cannot be withdrawn. This therefore prevents subsequently withdrawal of piston and therefore prevents re-use of the syringe.

The World Health Organization (WHO) requires all syringes used for vaccine should be self-destructive and cannot be reused. Meanwhile, the shape-destructed area of this syringe is very large, and the shape-destructed storage container is also enlarged at the same time thereby increasing the cost. Based on the above defects, a further improvement in the syringe is required.

In order to resolve the above defects, the present invention provides a self-destructive syringe with drug dissolved for once. The self-destructive syringe with drug dissolved for once according to the present invention is low at the production cost and convenient to operate, while the backstreaming of blood can be also observed, and the space of the backstreaming of blood is less than 0.03 ml, and it cannot be reused for dissolving drug two times strictly.

### SUMMARY OF THE INVENTION

The object of the present invention provides a self-destructive syringe for once suitable for dissolving the drug and injecting one time. More specifically, after the powdered drug needs to be diluted firstly, and then the liquid drug is extracted and injected, that is, the diluent is drawn and then injected into the powdered drug firstly, after diluting the liquid drug is re-drawn and then injected into human body using the syringe, which is the whole procedure. Thereafter the syringe cannot be reused.

To resolve the above reasonable whole procedure, the self-destructive syringe of the present invention is realized by the technical features as specified in claim 1.

The self-destructive syringe with drug dissolved for once as described above, characterized in that: a break part 15 is located at the front end of the slot of the core rod 3.

The self-destructive syringe with drug dissolved for once as described above, characterized in that: the contrast between the color of sealing plug 12 and the color of graduation lines 16 on exterior wall of the cylinder 2 is strong, and the color of the sealing plug are light blue, light green or white.

The self-destructive syringe with drug dissolved for once according to the present invention compared with the prior art has the beneficial effects as follows:
1. The construction of the syringe is simple, thus the production cost is lower;
2. It is convenient for operation when the syringe is used in the clinic. The backstreaming of blood can be observed, and the space of the backstreaming of blood is less than 0.03 ml, so that it meets the clinical requirement;
3. Since for the first time according to the practically required volume of the diluent the diluent is drawn to inject into the powder, and then for the second time the drug liquid is drawn to the specified dose to perform injection, so that it meets a requirement that the powder used in the clinic needs to be diluted;
4. Since the syringe cannot be re-drawn after the injection is completed, so that it is achieved that the syringe cannot be reused to dissolve the drug two times and can be self-destructed.

### BRIEF DESCRIPTION OF THE DRAWINGS

FIG. 1 is a schematic drawing of the self-destructive syringe of the present invention;
FIG. 2 is a schematic drawing showing the needle is adhered to the cylinder and the graduation lines are marked on the exterior wall of the cylinder;
FIG. 3 is a schematic drawing of the core rod;
FIG. 4 is a schematic drawing of the non-return element;
FIG. 5 is a schematic drawing of a operation procedure of the self-destructive syringe of the present invention, which includes eight sub-Figs. A, B, C, D, E, F, G and H;

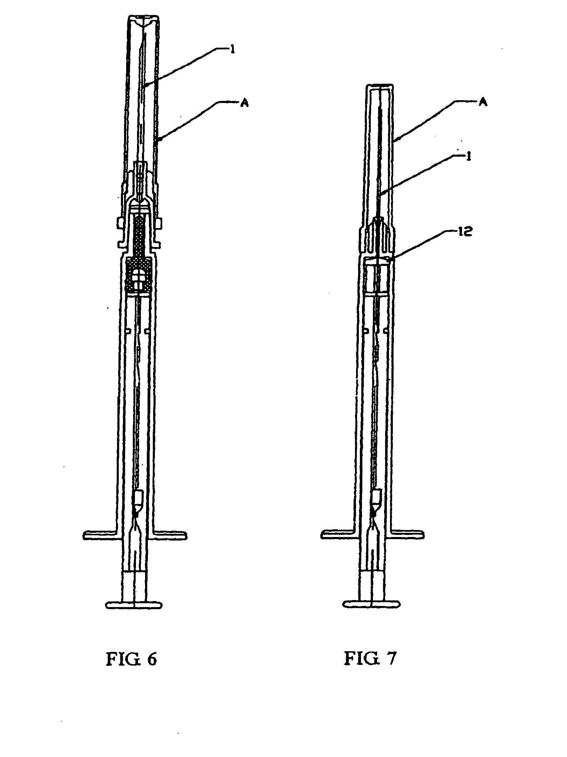

FIG. 6 is schematic drawing showing a self-destructive syringe of another example; and
FIG. 7 is schematic drawing showing a self-destructive syringe of another example and indicating the sealing plug is integrated in the core rod.

In the drawings, A: cap, 1: needle, 2: cylinder, 3:core rod, 4: the first ratchet groove, 5:the second ratchet groove, 6: the third ratchet groove, 7:the fourth ratchet groove, 8: mounting hole, 9: non-return element, 10: non-return pike, 11: ratchet, 12: sealing plug, 13,14: reciprocating part, 15: break part, and 16: graduation lines.

### DETAILED DESCRIPTION OF THE PREFERRED EMBODIMENTS

The invention will be further illustrated by an embodiment of the present invention and the accompanying drawings, with reference to the accompanying drawings.

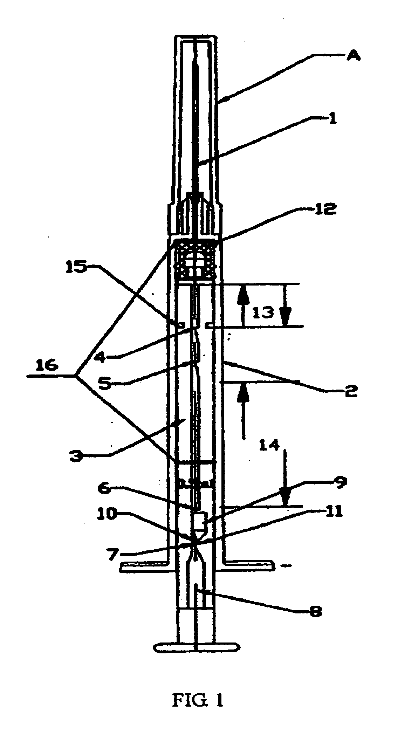

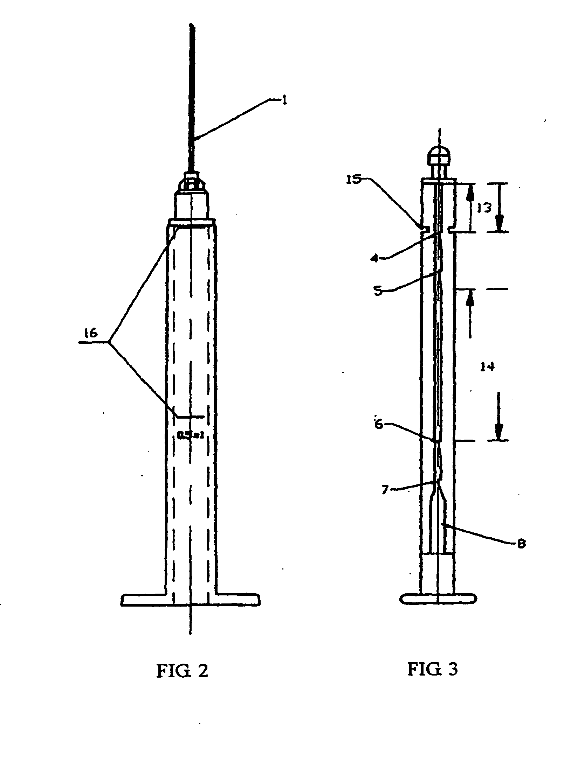

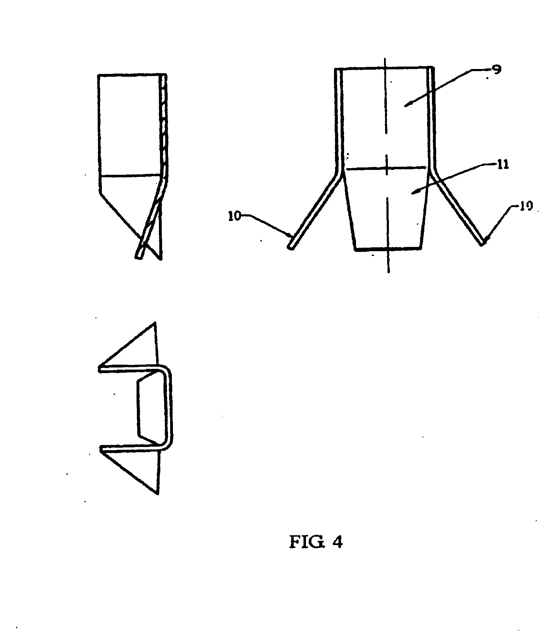

The self-destructive syringe with drug dissolved for once of the present invention comprises: a cap A, a needle 1, a cylinder 2, a core rod 3, a non-return element 9, and a sealing plug 12; a first ratchet groove 4, a second ratchet groove 5, a third ratchet groove 6 and a fourth ratchet grove 7 are located on the core rod 3; a break part 15 is located at the front end of the slot in the core rod; and a mounting hole 8 is located at the tail end of the core rod (see Fig. 3); a non-return pike 10 and a ratchet 11 are located on the non-return element 9 (see Fig. 4); when mounting the non-return element 9 is mounted between the third and the fourth ratchet grooves through a mounting hole 8, the core rod and the sealing plug 12 which is sleeved on the head of the core rod are both located within the cylinder 2 (see Fig.1).

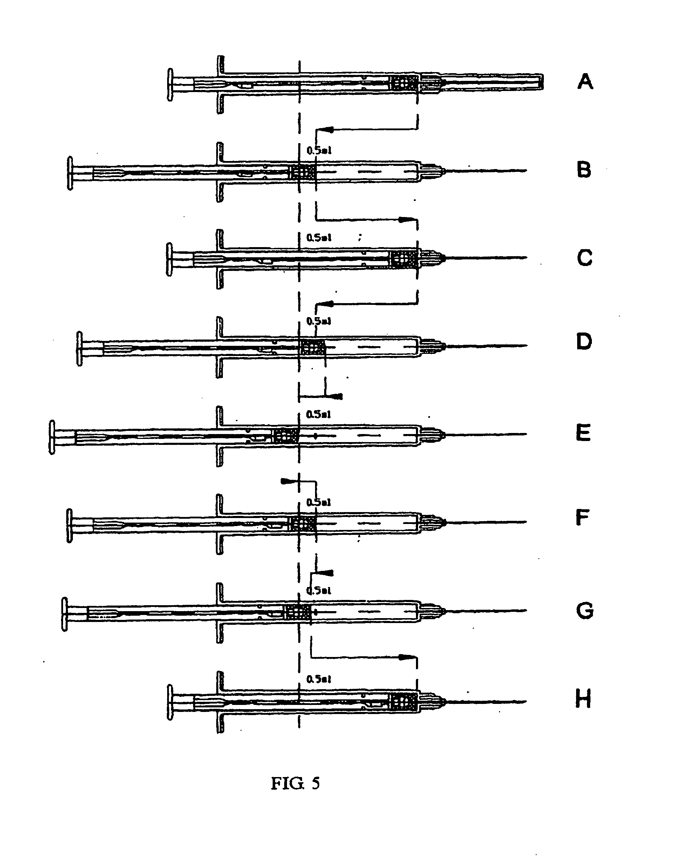

When using the syringe, the cap A is removed, the first step is drawing up 0.5 ml of the diluent into the syringe (for example, a 0.5 ml syringe). The graduation lines marked on the exterior wall of the cylinder is black, there is a distinctive graduation line marking 0.5ml of volume, while the color of the sealing plug is light blue, light green or white color, so that it is visible when the sealing plug passes through the graduations. The backward pulling is stopped when the head of the sealing plug reaches at 0.5 ml graduation line, at this moment the non-return element 9 has been already passed through the third ratchet groove on the core rod and but not passed through the second ratchet groove on the core rod. Then 0.5 ml of the diluent is injected into the drug powder to dilute. During the process of the injection, since the non-return element 9 has passed through the third ratchet groove when the diluent is drawn, the ratchet 11 on the non-return element 9 has engaged the third ratchet groove on the core rod, and they are pushed forward together during the later period of the injection (see sub-Figs. B and C of Fig. 5). After the dilution is completed, about 0.4 ml of the liquid drug is re-drawn, at this moment the ratchet 11 on the non-return element 9 has entered into the second ratchet groove on the core rod, and at this moment thus the self-destructive function becomes effective (see sub-Fig. D of Fig. 5). Then the operator continues drawing the liquid drug to make the cord rod reached at the top, at this moment the ratchet 11 on the non-return element 9 enters into the first ratchet groove on the core rod, and at the same time the liquid drug extraction is completed (see sub-Fig. E of Fig. 5). Then the air in the cylinder is expelled so that the volume of the liquid drug is up to the point of 0.5 ml in accordance with the injection requirement (see sub-Fig. F of Fig. 5). At this moment, since the length of the reciprocating part 13 on the core rod is 0.5 mm - 2 mm longer than that of the non-return element, the core rod of the syringe can be drawn backwards arbitrarily to observe whether the backstreaming of blood occurs after injecting into human body (see sub-Fig. G of Fig. 5). If the backstreaming of blood does not occur, the operator may inject directly (see sub-Fig. H of Fig. 5). At this moment, the whole procedure of the injection is completed, and the core rod of the syringe cannot be drawn any more, because two non-return pikes 10 on the non-return element 9 are all pierced on the interior wall of the cylinder 2, and thus the core rod is unable to be drawn. The core rod 3 will be broken immediately if on the core rod the break part 15 is rotated.

## Claims

1. A self-destructive syringe, comprising:
a cylinder (2), a needle (1) being adhered on the cylinder (2) or a needle holder with a needle (1) being sleeved on the cylinder (2), the exterior wall of the cylinder (2) being marked with graduation lines (16) in black color or dark colors;
a core rod (3) being located in the cylinder (2);
**characterized in that**:
a non-return element (9) being mounted in a central slot of the core rod (3) and being located within the cylinder (2);
a sealing plug (12) being mounted on the core rod (3), the sealing plug (12) being located on the front end of the core rod (3) and being located within the cylinder (2);
wherein
1) a first reciprocating area (13), a first ratchet groove (4), a second ratchet groove (5), a second reciprocating area (14), a third ratchet groove (6), a fourth ratchet groove (7) and a rectangular mounting hole (8) are sequentially arranged and located in and along the central slot of the core rod (3)
from the front end to the tail end thereof, the first reciprocating area (13) being the area form the front end to the first ratchet groove (4), the second reciprocating area (14)being the area from the second ratchet groove (5) to the third ratchet groove (6);
2) the mounting hole (8) is located between the fourth ratchet groove (7) and the tail end of the core rod (3);
3) the length of the first reciprocating area (13) is 0.5 - 2 mm longer than that of the non-return element (9) ;
4) a non-return pike (10) and a ratchet (11) are located on the non-return element (9), the non-return element (9) is made of elastic steel sheet, the non-return element (9) is mounted in the slot of the core rod (3) through the mounting hole (8) and is initially mounted between the third ratchet groove (6) and the fourth ratchet groove (7).

2. The self-destructive syringe according to claim 1,
**characterized in that**: a break part (15) is located at the front end of the slot of the core rod (3).

3. The self-destructive syringe according to claim 1,
**characterized in that**: the contrast between the color of the sealing plug (12) and the color of graduation lines (16) on the exterior wall of the cylinder (2) is strong, and the color of the sealing plug (12) are light blue, light green or white.

## Patentansprüche

1. Sich selbst zerstörende Spritze, umfassend:
einen Zylinder (2), eine auf dem Zylinder (2) haftende Nadel (1) oder ein Nadelhalter mit einer Nadel (1), die auf den Zylinder (2) gesteckt ist, wobei auf die Außenwand des Zylinders (2) Skaleneinteilungslinien (16) in schwarz oder dunklen Farben gezeichnet sind;
ein in dem Zylinder (2) angeordnete mittlere Kolbenstange (3);
**dadurch gekennzeichnet, dass**:
ein Rückzugsperrelement (9), das in der Aussparung der mittleren Kolbenstange (3) befestigt ist und innerhalb des Zylinders (2) angeordnet ist;
ein Verschlussstopfen (12), der auf der mittleren Kolbenstange (3) befestigt ist, wobei der Verschlussstopfen (12) auf dem vorderen Ende der mittleren Kolbenstange (3) angeordnet ist und innerhalb des Zylinders (2) angeordnet ist;
wobei1) ein erster sich hin und her bewegender Teil (13), eine erste Sperrnut (4), eine zweite Sperrnut (5), ein zweiter sich hin und her bewegender Teil (14), eine dritte Sperrnut (6), eine vierte Sperrnut (7) und ein rechteckiges Befestigungsloch (8) die aufeinanderfolgend angeordnet sind und in und entlang der Aussparung der mittleren Kolbenstange (3) angebracht ist von dem vorderen Ende bis zum Endstück davon, der erste sich hin und her bewegende Teil (13), der das Teil vom vorderen Ende bis zu der ersten Sperrnut (4) ist, der zweite sich hin und her bewegende Teil (14), der das Teil von der zweiten Sperrnut (5) bis zu der dritten Sperrnut (6) ist;
2) das rechteckige Befestigungsloch (8) angebracht zwischen der vierten Sperrnut (7) und dem Endstück der mittleren Kolbenstange (3);
3) die Länge des ersten sich hin und her bewegenden Teils (13) 0,5 mm - 2 mm länger ist als die des Rückzugsperrelements (9);
4) ein Rückzugsperrhaken (10) und eine Sperrvorrichtung (11) auf dem Rückzugsperrelement (9) angeordnet sind, das Rückzugsperrelement (9) aus elastischem Stahlblech hergestellt ist, das Rückzugsperrelement (9) in der Aussparung der mittleren Kolbenstange (3) durch das Befestigungsloch (8) befestigt ist und zwischen der dritten Sperrnut (6) und der vierten Sperrnut (7) am Anfang befestigt ist.

2. Sich selbst zerstörende Spritze, nach Anspruch 1, **dadurch gekennzeichnet, dass**: ein Sollbruchteil (15) an dem vorderen Ende der Aussparung der mittleren Kolbenstange (3) angeordnet ist.

3. Sich selbst zerstörende Spritze nach Anspruch 1, dadurch charakterisiert, dass es einen starken Kontrast zwischen der Farbe des Verschlussstopfens (12) und der Farbe der Skaleneinteilungslinien (16) auf der Außenwand des Zylinders (2) gibt und die Farbe des Verschlusstopfens (12) hellblau, hellgrün oder weiß ist.

## Revendications

1. Seringue autodestructrice comprenant :
un cylindre (2), une aiguille (1) qui adhère au cylindre (2) ou un support d'aiguille avec une aiguille (1) qui glisse sur le cylindre (2), la paroi extérieure du cylindre (2) étant marquée de traits de graduation (16) en noir ou en couleurs sombres ;
une tige centrale (3) étant située dans le cylindre (2) ;
**caractérisée en ce que** :
un élément de non retour (9) étant monté dans la fente de la tige centrale (3) et étant situé au sein du cylindre (2) ;
un bouchon d'étanchéité (12) qui est monté sur la tige centrale (3), le bouchon d'étanchéité (12) étant situé sur l'extrémité avant de la tige centrale (3) et étant situé au sein du cylindre (2) ;
dans lequel :
1) une première partie à va-et-vient (13), une première rainure d'encliquetage (4), et une deuxième rainure d'encliquetage (5), une deuxième partie à va-et-vient (14), une troisième rainure d'encliquetage (6), une quatrième rainure d'encliquetage (7) et un orifice de montage rectangulaire (8) qui sont montées et situées séquentiellement dans et de long de la fente de la tige centrale (3) de l'extrémité avant à l'extrémité dernière de cela, la première partie à va-et-vient (13) est la partie de l'extrémité avant à la première rainure d'encliquetage (4), la deuxième partie à va-et-vient (14) être la partie de la deuxième rainure d'encliquetage (5) à la troisième rainure d'encliquetage (6);
2) l'orifice de montage rectangulaire (8) est localisé entre la quatrième rainure d'encliquetage (7) et l'extrémité en arrière de la tige centrale (3] ;
3) la longueur de la partie à va-et-vient (13) est de 0,5 mm à ~ 2 mm plus longue que celle de l'élément de non retour (9) ;
4) une saillie de non retour (10) et un cliquet (11) sont situés sur l'élément de non retour (9), l'élément de non retour (9) est réalisé en feuille d'acier élastique, l'élément de non retour (9) est monté dans la fente de la tige centrale (3) à travers l'orifice de montage (8) et est monté entre la troisième rainure d'encliquetage (6) et la quatrième rainure d'encliquetage (7).

2. Seringue autodestructrice selon la revendication 1, **caractérisée en ce que**: une partie de cassure (15) est située à l'extrémité avant de la fente de la tige centrale (3).

3. Seringue la revendication 1, **caractérisée en ce que**: le contraste entre la couleur du bouchon d'étanchéité (12) et la couleur des traits de graduation (16) sur la paroi extérieure du cylindre (2) est fort, et la couleur du bouchon d'étanchéité (12) est bleu clair, vert clair ou blanc.
